(19) Europäisches Patentamt
European Patent Office
Office européen des brevets

(11) EP 0 930 301 B1

(12) EUROPÄISCHE PATENTSCHRIFT

(45) Veröffentlichungstag und Bekanntmachung des Hinweises auf die Patenterteilung:
**28.07.2004 Patentblatt 2004/31**

(21) Anmeldenummer: **99103925.6**

(22) Anmeldetag: **18.12.1991**

(51) Int Cl.[7]: **C07D 213/61**, C07D 213/64, C07D 213/65, C07D 401/14, C07D 401/12, C07D 405/04, C07D 405/12, C07D 417/04, C07D 417/12, C07F 5/02, C07F 7/10

# (54) 2-Fluorpyridine, Verfahren zu ihrer Herstellung und ihre Verwendung in Flüssigkristallmischungen

2-Fluoropyridine, process for producing it and its use in liquid crystal mixtures

2-Fluoropyridine, son procédé de production et son utilisation dans des mélanges de cristaux liquides

(84) Benannte Vertragsstaaten:
**AT BE CH DE DK ES FR GB GR IT LI NL SE**

(30) Priorität: **19.12.1990 DE 4040575**
**09.04.1991 DE 4111461**

(43) Veröffentlichungstag der Anmeldung:
**21.07.1999 Patentblatt 1999/29**

(62) Dokumentnummer(n) der früheren Anmeldung(en) nach Art. 76 EPÜ:
**92901407.4 / 0 563 146**

(73) Patentinhaber: **Aventis Research & Technologies GmbH & Co. KG**
**65926 Frankfurt am Main (DE)**

(72) Erfinder:
- **Schlosser, Hubert, Dr.**
  **61479 Glashütten (DE)**
- **Wingen, Rainer, Dr.**
  **65795 Hattersheim (DE)**
- **Illian, Gerd, Dr.**
  **50374 Erfstadt (DE)**
- **Escher, Claus, Dr.**
  **64367 Mühltal (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**WO-A-91/04248** **WO-A-91/04249**
**WO-A-92/09576** **DE-A- 3 346 175**

- **CHEMICAL ABSTRACTS, vol. 109, no. 16, 17. Oktober 1988 Columbus, Ohio, US; abstract no. 139705w, M.Y. KOK ET AL.: "Orientational order of biaxial solutes in nematic phases. Size and shape effects" Seite 721; Spalte 2; XP002900429 & LIQ. CRYST. , Bd. 3, Nr. 4, 1988, Seiten 485-505,**
- **CHEMICAL ABSTRACTS, vol. 92, no. 12, 24. März 1980 Columbus, Ohio, US; abstract no. 101895a, SURYAPRAKASH, N. ET AL.: "NMR spectra of 2-fluoropyridine in nematic liquid crystals" Seite 513; Spalte 2; XP002900430 & CURR. SCI., Bd. 49, Nr. 1, 1980, Seiten 1-3,**
- **CHEMICAL ABSTRACTS, vol. 86, no. 1, 3. Januar 1977 Columbus, Ohio, US; abstract no. 4353r, DUCHENE, M. ET AL.: "Structures of some fluorinated pyridines from the analysis of NMR spectra of nematic solutions" Seite 378; Spalte 1; XP002900431 & J. MAGN. RESON., Bd. 22, Nr. 2, 1976, Seiten 207-218,**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die ungewöhnliche Kombination von anisotropem und fluidem Verhalten der Flüssigkristalle hat zu ihrer Verwendung in elektro-optischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelberechnung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Auf besonderes Interesse sind in den letzten Jahren ferroelektrische flüssigkristalline Mischungen (FLC-Mischungen) gestoßen (siehe z.B. Lagerwall et al. "Ferroelectric Liquid Crystals for Display", SID Symposium, October Meeting 1985, San Diego, Ca. USA).

Für die praktische Verwendung von ferroelektrischen Flüssigkristallen in elektrooptischen Anzeigen werden chirale, geneigt-smektische Phasen wie z.B. $S_C$-Phasen benötigt [ siehe R.B. Meyer, L.Liebert, L. Strzelecki und P.Keller, J. Physique <u>36</u>, L-69 (1975)] , die über einen großen Temperaturbereich stabil sind. Dieses Ziel kann man mit Verbindungen erreichen, die selbst solche Phasen, z.B. $S_C$-Phasen, ausbilden oder aber, indem man nicht chirale geneigtsmektische Phasen ausbildende Verbindungen mit optisch aktiven Verbindungen dotiert [ siehe M. Brunet, Cl. Williams, Ann. Phys. <u>3</u>, 237 (1978)].

Bei der Verwendung ferroelektrischer Flüssigkristallmischungen in elektro-optischen Bauelementen ist eine einheitliche planare Orientierung der Flüssigkristalle notwendig, um ein hohes Kontrastverhältnis zu erzielen. Es hat sich gezeigt, daß sich eine einheitliche planare Orientierung in der $S_C$-Phase erreichen läßt, wenn die Phasenfolge der Flüssigkristalllmischung mit abnehmender Temperatur lautet: Isotrop→nematisch→smektisch A→smektisch C (siehe z.B. K. Flatischler et al., Mol. Cryst. Sig. Cryst. 131, 21 (1985); T. Matsumoto et al., p. 468-470, Proc. of the 6th Int. Display Research Conf., Japan Display, 30. September - 2. October 1986, Tokyo, Japan; M.Murakami et al., ibid. p. 344-347).

Für ferroelektrische (chiral smektische) Flüssigkristallmischungen muß zusätzlich die Bedingung erfüllt sein,. daß die Ganghöhe (pitch) der Helix in der $S^*_C$-Phase groß, d. h. größer als 5 µm, und in der N*-Phase sehr groß, d. h. größer als 10 µm bzw. unendlich ist.

Die optische Schaltzeit τ[µs] ferroelektrischer Flüssigkristallsysteme, die möglichst kurz sein soll, hängt von der Rotationsviskosität des Systems γ[mPas], der spontanen Polarisation $P_s$[nC/cm$^2$] und der elektrischen Feldstärke E[V/m] ab nach der Beziehung

$$\tau \approx \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie Δn, vorzugsweise ≈ O,13, und eine geringe positive oder vorzugsweise negative dielektrische Anisotrophie Δε verlangt (siehe S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Einzelne dieser Komponenten und auch bestimmte Mischungen sind bereits aus dem Stand der Technik bekannt. Da aber die Entwicklung, insbesondere von ferroelektrischen Flüssigkristallmischungen, noch in keiner Weise als abgeschlossen betrachtet werden kann, sind die Hersteller von Displays an unterschiedlichen Mischungen interessiert. Dieses u.a. auch deshalb, weil erst das Zusammenwirken der flüssigkristallinen Mischungen mit den einzelnen Bauteilen der Anzeigevorrichtungen bzw. der Zellen (z.B. der Orientierungsschicht) Rückschlüsse auf die Qualität auch der flüssigkristallinen Mischungen zuläßt.

In EP-B 0 158 137 werden 4-Fluorpyrimidine als Verbindungen und als Mischungskomponenten allgemein

beschrieben. Sie weisen jedoch keine oder nur eine geringe Tendenz zur Ausbildung smektischer Phasen auf und finden daher Einsatz in nematischen Mischungen.

In DE-A 40 29 165 sowie in DE-A 40 30 582 werden 4-Fluorpyrimidine als Komponenten für ferroelektrische Flüssigkristallmischungen vorgestellt.

Desweiteren ist bekannt, daß Mono- und Difluorphenylverbindungen als Komponenten von Flüssigkristallmischungen verwendet werden können (JP-A 2169-537; V.Reiffenrath, The Twelfth International Liquid Crystal Conference, Freiburg, 15.-19.August 1988). Diese Verbindungen haben jedoch zum Teil keine $S_C$-Phase. Ferner treten aufgrund fluorophober Wechselwirkungen häufig Mischbarkeitsprobleme mit strukturell unterschiedlichen Mischungskomponenten, z. B. Phenylpyrimidinen auf.

Pyridinderivate zeigen ebenfalls flüssigkristallines Verhalten unter Ausbildung einer $S_C$-Phase (T.Geelhaar, 1st International Symposium on Ferroelectric Liquid Crystal, Arcachon, 2.-23. September 1987; US 4,952,335). Eine in diesen Verbindungen häufig auftretende $S_I$-Phase beeinträchtigt jedoch deren Verwendung in ferroelektrischen Flüssigkristallmischungen.

DE-A-33 46 175 betrifft Pyridylthiophene für Flüssigkristallmischungen.

Die vorliegende Erfindung betrifft neue 2-Fluorpyridin-Derivate und deren Verwendung als Komponenten für ferroelektrische Flüssigkristallmischungen, wobei man mindestens ein 2-Fluorpyridin der allgemeinen Formel (I) als Komponente in einer Flüssigkristallmischung einsetzt.

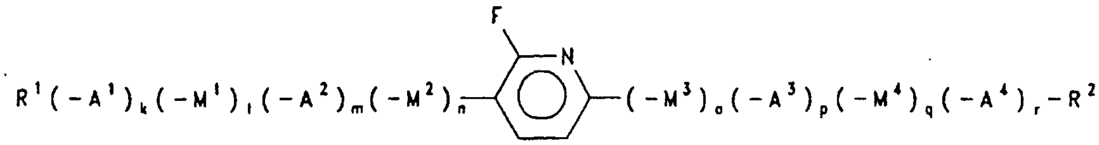

(I)

Die Symbole haben hierbei folgende Bedeutung:

$R^1$, $R^2$ unabhängig voneinander H,F,Cl,CN,-$CF_3$,-$OCF_3$,-$OCHF_2$ oder geradkettiges oder verzweigtes (mit oder ohne asymmetrisches C-Atom) Alkyl mit 1 bis 16 Atomen, wobei auch eine oder zwei nicht benachbarte -$CH_2$-Gruppen durch -O-,-CO-,-CO-O-,-O-CO-, -O-CO-O-,-CH=CH-,-C≡C-, oder -Si($CH_3$)$_2$- ersetzt sein können, und wobei auch ein oder mehrere H-Atome des Alkylrestes durch F substituiert sein können, oder eine der nachfolgenden chiralen Gruppen:

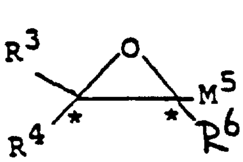 , 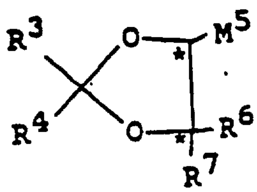 , 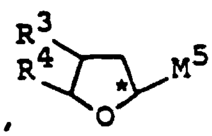 , 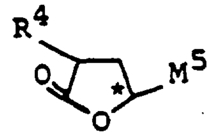 ,

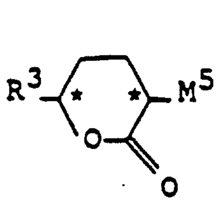 , 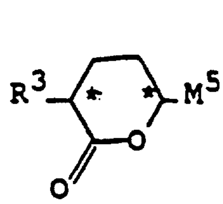 , 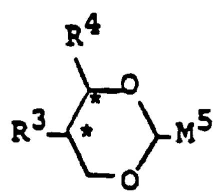 , 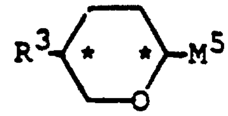

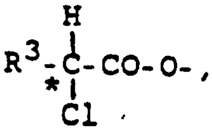, 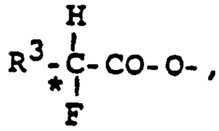, 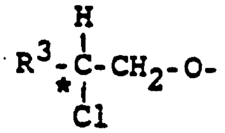, 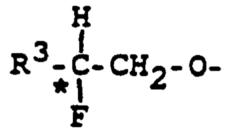,

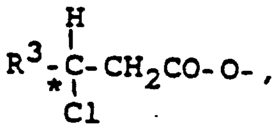, 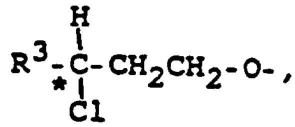,

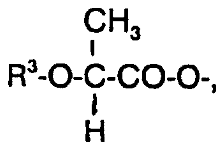, 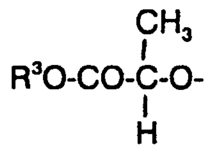

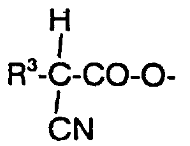, 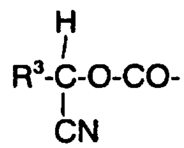

$A^1$, $A^2$, $A^3$, $A^4$ sind gleich oder verschieden 1,4-Phenylen, Pyrazin-2,5-diyl, Pyridazin--3,6-diyl, Pyridin-2,5-diyl, Pyrimidin-2,5-diyl, wobei ein oder zwei H-Atome durch F ersetzt sein können, trans-1,4-Cyclohexylen, bei dem ein oder zwei H-Atome durch CN ersetzt sein können, (1,3,4)-Thiadiazol-2,5-diyl, 1,3-Dioxan-2,5-diyl, Naphthalin-2.6-diyl, Bicyclo[2.2.2.]octan-1,4diyl oder 1,3-Dioxaborinan-2,5-diyl;

$M^1$, $M^2$, $M^3$, $M^4$ gleich oder verschieden -O-, -CO-, -CO-O-, -O-CO-, -O-CO-O-, $-CH_2-O-$, $-O-CH_2-$, $-CH_2-CH_2-$, -CH=CH- oder -C≡C-;

$R^3$, $R^4$, $R^6$, $R^7$ unabhängig voneinander H oder geradkettiges oder verzweigtes Alkyl mit 1 bis 16 C-Atomen oder $R^3$ und $R^4$ zusammen auch $-(CH_2)_4-$ oder $-(CH_2)_5-$, wenn als Substituenten an ein Dioxolan System gebunden.

$M^5$ $-CH_2-O-$, -CO-O-, $-O-CH_2-$, -O-CO- oder eine Einfachbindung k, l, m, n, o, p, q, r Null oder Eins, unter der Bedingung, daß die Summe k+m+p+r kleiner 4 und größer Null ist,

mit der Maßgabe, daß eine der beiden Gruppen
$R^1$ und $R^2$ die Gruppe

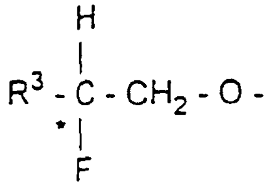

ist.

In einer bevorzugten Ausführung der Erfindung haben die Symbole folgende Bedeutung:

$R^1$, $R^2$ unabhängig voneinander H, F, CN oder geradkettiges oder verzweigtes (mit oder ohne asymmetrisches C-Atom) Alkyl mit 1 bis 16 C-Atomen, wobei auch eine $-CH_2$-Gruppe durch -O-, -CO-, -CO-O-, -O-CO-, -O-CO-O-, -CH=CH-, -C≡C-,
oder $-Si(CH_3)_2$- ersetzt sein können, oder eine der nachfolgenden chiralen Gruppen:

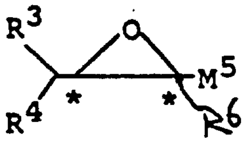 , 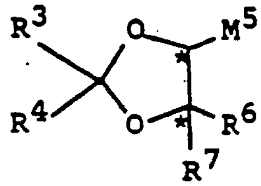 , 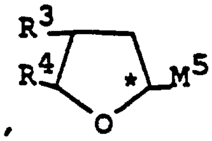 , 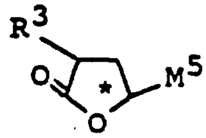 ,

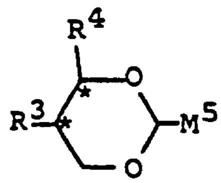 ,

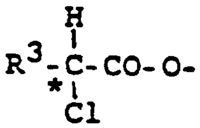 , 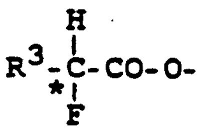 , 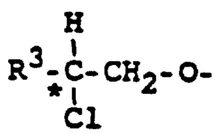 , 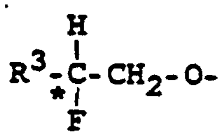 ,

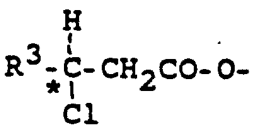 , 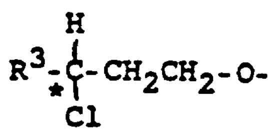 ,

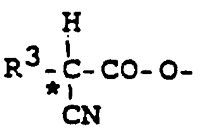 , 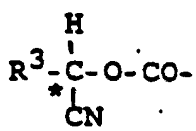

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome durch F ersetzt sein können, trans-1,4-Cyclohexylen, (1,3,4)-Thiadiazol-2,5-diyl, 1,3-Dioxan-2,5-diyl Naphthalin-2,6-diyl, Bicyclo[2.2.2]octan-1,4-diyl oder 1,3-Dioxaborinan-2,5-diyl;

$M^1$, $M^2$, $M^3$, $M^4$ gleich oder verschieden -O-, -CO-, -CO-O-, -O-CO-, $-CH_2$-O-, -O-$CH_2$, $-CH_2-CH_2$-, -CH=CH- oder -C≡C-;

$R^3$, $R^4$, $R^6$, $R^7$ unabhängig voneinander H oder geradkettiges oder verzweigtes Alkyl mit 1 bis 10 C-Atomen oder $R^3$ und $R^4$ zusammen auch $-(CH_2)_4$-oder $-(CH_2)_5$- wenn als Substituenten an ein Dioxolan-System gebunden;

$M^5$ $-CH_2$-O-, -CO-O-, -O-$CH_2$-, -O-CO- oder eine Einfachbindung.

Ferner werden 2-Fluorpyridin-Derivate bevorzugt, bei denen die Symbole folgende Bedeutung haben:

$R^1$, $R^2$ unabhängig voneinander H, F, CN oder geradkettiges oder verzweigtes (mit oder ohne asymmetrisches C-Atom) Alkyl mit 1 bis 16 C-Atomen, wobei auch eine -$CH_2$-Gruppe durch -O-, -CO-, -CO-O-, -O-CO-, -CH=CH-, oder -Si $(CH_3)_2$- ersetzt sein können, oder eine der nachfolgenden chiralen Gruppen:

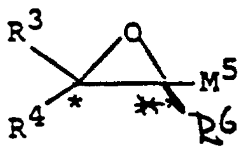 , 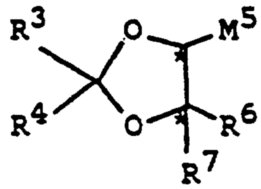 , 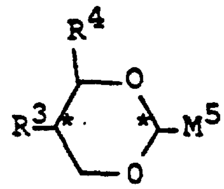 ,

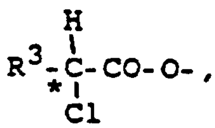 , 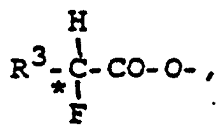 ,

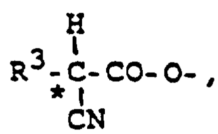 ,

$A^1$, $A^2$, $A^3$, $A^4$ gleich oder verschieden 1,4-Phenylen, Pyrazin-2,5-diyl, Pyridazin-3,6-diyl, Pyridin-2.5-diyl, Pyrimidin-2,5-diyl, bei dem ein oder zwei H-Atome durch F ersetzt sein können, trans-1,4-Cyclohexylen, 1,3-Dioxan-2,5-diyl, Naphthalin-2,6-diyl oder 1,3-Dioxaborinan-2,5-diyl;

$M^1$, $M^2$, $M^3$, $M^4$ gleich oder verschieden -O-, -CO-O-, -O-CO-, -O-$CH_2$, -$CH_2$-$CH_2$- oder -CH=CH-;

$R^3$, $R^4$, $R^6$, $R^7$ unabhängig voneinander H oder geradkettiges oder verzweigtes Alkyl mit 1 bis 10 C-Atomen oder $R^3$ und $R^4$ zusammen auch -$(CH_2)_4$- oder -$(CH_2)_5$-, wenn als Substituenten an ein Dioxolan-System gebunden;

$M^5$ -$CH_2$-O-, -CO-O-, -O-$CH_2$-, -O-CO- oder eine Einfachbindung.

Insbesondere bevorzugt ist ein 2-Fluorpyridin, bei dem $R^1$, $R^2$ unabhängig voneinander H oder Alkyl mit 1 bis 16 C-Atomen sind, wobei auch eine -$CH_2$-Gruppe durch -O-, -CO-O- oder -O-CO- ersetzt sein kann, oder die chirale Gruppe

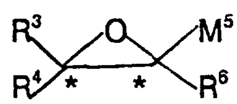

$A^1$, $A^2$, $A^3$, $A^4$ sind gleich oder verschieden 1,4-Phenylen, Pyrazin-2,5-diyl, Pyridazin-3,6-diyl, Pyridin-2,5-diyl, Pyrimidin-2,5-diyl, bei dem ein oder zwei H-Atome durch F ersetzt sein können, trans-1,4-Cyclohexylen, 1,3-Dioxan-2,5-diyl, Naphthalin-2,6-diyl oder 1,3 Dioxaborinan-2,5-diyl;

$M^1$, $M^2$, $M^3$, $M^4$ sind gleich oder verschieden -O-, -CO-O-, -O-CO-, -$OCH_2$- oder -$CH_2$-O-;

$R^3$, $R^4$, $R^6$ sind unabhängig voneinander H oder ein geradkettiges Alkyl mit 1 bis 10 C-Atomen;

$M^5$ ist -$CH_2$-O- oder -CO-O-.

Die erfindungsgemäßen Verbindungen sind chemisch und photochemisch stabil. Sie verfügen über niedrige Schmelzpunkte und im allgemeinen breite flüssigkristalline Phasen, insbesondere breite nematische, smektische A und smektische C-Phasen.

Aus flüssigkristallinen Verbindungen mit diesem Strukturelement lassen sich sowohl ferroelektrische Mischungen als auch nematische oder auch chiral nematische Mischungen herstellen, die für die Anwendung in elektrooptischen oder vollständig optischen Elementen, z.B. Anzeigeelementen, Schaltelementen, Lichtmodulatoren Elementen zur Bildbearbeitung, Signalverabeitung oder allgemein im Bereich der nichtlinearen Optik geeignet sind.

Ein besonderer Vorteil dieser Komponenten beruht auf ihrer negativen dielektrischen Anisotropie, die besonders günstig für die AC-Feld-Stabilisationen bei der Anwendung von ferroelektrischen Flüssigkristallen ist (siehe z.B. 1983 SID report von AT & T, JP-A 245142/1986, JP-A 246722/1986, JP-A 246723/1986).

Außerdem benötigt man für Displays, die sich vom ECB- bzw. CSH-Prinzip ableiten (siehe z.B. S. Yamauchi et al., SID 1989 Digest, Seite 378 oder M.F. Schiekel und K. Fahrensohn, Appl. Phys. Lett., S. 391 (1971)), nematische Mischungen mit möglichst großer negativer dielektrischer Anisotropie. Die Fluorpyridin-Derivate sind aufgrund ihrer breiten nematischen Phase, ihrer niedrigen Viskosität und ihrer großen negativen dielektrischen Anisotropie für diese nematischen Mischungen als Hauptkomponente oder als Beimischung geeignet.

Eine weitere Lösung der gestellten Aufgabe ist eine flüssigkristalline, insbesondere eine ferroelektrische flüssigkristalline Mischung, die mindestens eine Verbindung der allgemeinen Formel (I) enthält. Prinzipiell sind die genannten 2-Fluorpyridine jedoch ebenfalls als Komponenten für nematische Flüssigkristallmischungen sowie für den Einsatz in nematischen Displays geeignet.

Die Flüssigkristallmischungen bestehen im allgemeinen aus 2 bis 20, vorzugsweise 2 bis 15 Komponenten, darunter mindestens eine der erfindungsgemäß beanspruchten Verbindungen. Die anderen Bestandteile werden vorzugsweise ausgewählt aus den bekannten Verbindungen mit nematischen, cholesterischen und/oder geneigt- smektischen Phasen, dazu gehörten beispielsweise Schiff'sche Basen, Biphenyle, Pyridine, Thiadiazole, Difluorphenyle, Terphenyle, Phenylcyclohexane, Cyclohexylbiphenyle, Pyrimidine, Zimtsäureester, Cholesterinester sowie mehrkernige Ester von p-Alkylbenzoesäuren. Im allgemeinen liegen die im Handel erhältlichen Flüssigkristallmischungen bereits vor der Zugabe der erfindungsgemäßen Verbindung(en) als Gemische verschiedenere Komponenten vor, von denen mindestens eine mesogen ist.

Von dem oder den erfindungsgemäßen 2-Fluorpyridin-Derivaten enthalten die Flüssigkristallmischungen im allgemeinen 0,1 bis 70 Mol-%, bevorzugt 0,5 bis 50 Mol-%, insbesondere 1 bis 25 Mol-%.

Für die gebrauchsfertigen ferroelektrischen Flüssigkristallmischungen wurden die Werte für die spontane Polarisation $P_s$[nC/cm$^2$], den Kontrast K und die optische Schaltzeit $\tau$[µs] bestimmt, wobei alle Messungen bei einer Temperatur von 25°C vorgenomen wurden.

Die $P_s$-Werte werden nach der Methode von H. Diamant et al. (Rev. Sci. Instr., 28, 30, 1957) gemessen, wobei Meßzellen mit 2 µm Elektrodenabstand und geriebenem Polyimid als Orientierungsschicht verwendet werden. Zur Bestimmung von $\tau$ und K wird die Meßzelle auf dem Drehtisch eines Polarisationsmikroskops zwischen gekreuztem Analysator und Polarisator befestigt. Für die Bestimmung des Kontrastes (K) wird die Meßzelle durch Drehen so positioniert, daß eine Photodiode minimalen Lichtdurchgang anzeigt (Dunkelzustand). Die Mikroskop-Beleuchtung wird so geregelt, daß die Photodiode für alle Zellen die gleiche Lichtintensität anzeigt. Nach einem Schaltvorgang ändert sich die Lichtintensität (Hellzustand) und der Kontrast wird aus dem Verhältnis der Lichtintensitäten dieser Zustände berechnet.

Zur Bestimmung von r und des Schaltwinkels $\varnothing_{eff}$ wird durch Drehen des Tisches die Position des Tisches mit minimalem Lichtdurchgang für die beiden Schaltzustände in der Zelle bestimmt. Die Differenz der beiden Positionen am Drehtisch ist gleich dem doppelten effektiven Tiltwinkel. Mit Hilfe einer Photodiode erfolgt die Bestimmung der Schaltzeit $\tau$, indem die Anstiegszeit des Lichtsignals von 10 auf 90 % Signalhöhe gemessen wird. Die Schaltspannung besteht aus Rechteckpulsen und beträgt ± 10 V/µm.

Die Phasenumwandlungstemperaturen werden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | (N bzw. N*) |
| Smektisch-C | ($S_C$ bzw. $S_C$*) |
| Smektisch-A | ($S_A$ bzw. $S_A$*) |
| Kristallin | (X) |

erfolgt in °C und die Werte stehen zwischen den Phasenbezeichnungen in der Phasenfolge.

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Schalt- und Anzeigevorrichtungen (LC-Displays) weisen u.a. folgende Bestandteile auf: ein flüssigkristallines Medium, Trägerplatten (z.B. aus Glas oder Kunststoff), beschichtet mit transparenten Elektroden, mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie z.B. Dünnschichttransistoren (TFT) und Metall-Isolator-Metall-(MIM)-Elemente. Im Detail ist der Aufbau von Flüssigkristalldisplays bereits in einschlägigen Monographien beschrieben (z.B. E.

Kaneko, "Liquid Crystal TV Displays: Prinicples and Applications of Liquid Crystal Displays", KTK Scientific Publishers, 1987, Seiten 12-30 und 63-172).

Die Herstellung der erfindungsgemäßen Verbindungen kann durch die in den Schemata 1 bis 5 dargestellten Verfahren A und B erfolgen. Beide Verfahren unterscheiden sich prinzipiell dadurch, daß im Verfahren A ein Pyridinderivat, welches bereits die mesogene Grundstruktur einschließlich der Flügelgruppen enthält, durch geeignete Umsetzungen in Nachbarstellung zum Pyridin-Stickstoff fluoriert wird, während im Verfahren B ein bereits fluoriertes Pyridin durch geeignete Umsetzungen sukzessive mit weiteren mesogenen Einheiten und den Flügelgruppen versehen wird.

Die Erfindung beschreibt somit sowohl ein Verfahren zur Herstellung eines 2-Fluorpyridins der allgemeinen Formel (I) durch Fluorierung eines Pyridin-Derivates der Formel (A) am Pyridinring in 2-Position,

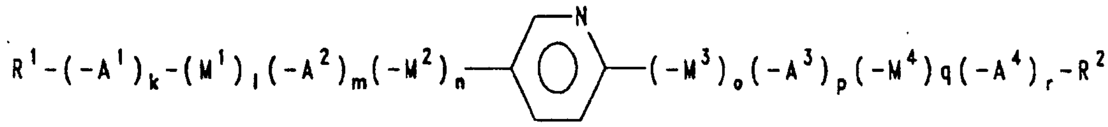

(A)

wie auch ein Verfahren, bei dem ausgehend von 2-Brom-6-fluorpyridin bzw. 2,6-Difluor-pyridin durch mehrstufige Umsetzung die Seitenketten

$R^1(-A^1)_k(-M^1)_l(-A^2)_m(-M^2)_n$- und

$-(M^3)_o-(A^3)_p-(M^4)_q-(A^4)_r-R^2$ in 3- bzw. 6-Position des Pyridinringes eingebracht werden.

Das in Schema 1 gezeigte Verfahren A geht von 2,5-disubstituierten Pyridinen der allgemeinen Formel (11) aus, die nach an sich literaturbekannten Methoden (z.B. C.S. Giam, J. Stout, Chem. Commun. 478 (1970); P. Doyle, R.R. Yates, Tetrahedron Lett. 3371 (1970); A.I. Pavljucenko et al., Z. Org. Chem. 22 (1986), 1061) dargestellt werden können. Die Pyridinderivate der Formel (11) lassen sich durch Umsetzung mit einer Percarbonsäure, z.B. Perameisensäure, Peressigsäure, Perbenzoesäure, 3-Chlorperbenzoesäure in das N-Oxid (Formel (III)) überführen. Die Verbindungen der Formel (III) können durch Behandlung mit einem Carbonsäureanhydrid und sich anschließender alkalischer Aufarbeitung in die Pyridone der Formel (IV) umgewandelt werden. Einen direkten Zugang zu den 2-Fluorpyridinderivaten der Formel (I) ausgehend von Pyridonen der Formel (IV) bietet die Umsetzung der letzteren mit einem Fluorierungsmittel wie z.B. Aminofluorosulfuranen (z.B. Diethylaminoschwefeltrifluorid) oder Schwefeltetrafluorid.

Alternativ können Pyridone der Formel (IV) unter Verwendung eines Halogenierungsmittels, wie z.B. Phosphortrichlorid, Phosphoroxytrichlorid, Phosphorpentachlorid und deren Brom- und Jod-Analoga, in die entsprechenden 2-Halogenpyridine der Formel (V) überführt werden. Der nucleophile Austausch der Halogensubstituenten gegen Fluor unter Zuhilfenahme eines Fluorid-Reagenzes wie z.B. Silberfluorid, Natriumfluorid, Kaliumfluorid oder Caesiumfluorid führt ebenfalls zu 2-Fluorpyridinen der Formel (I).

Schema 1

(Verfahren A)

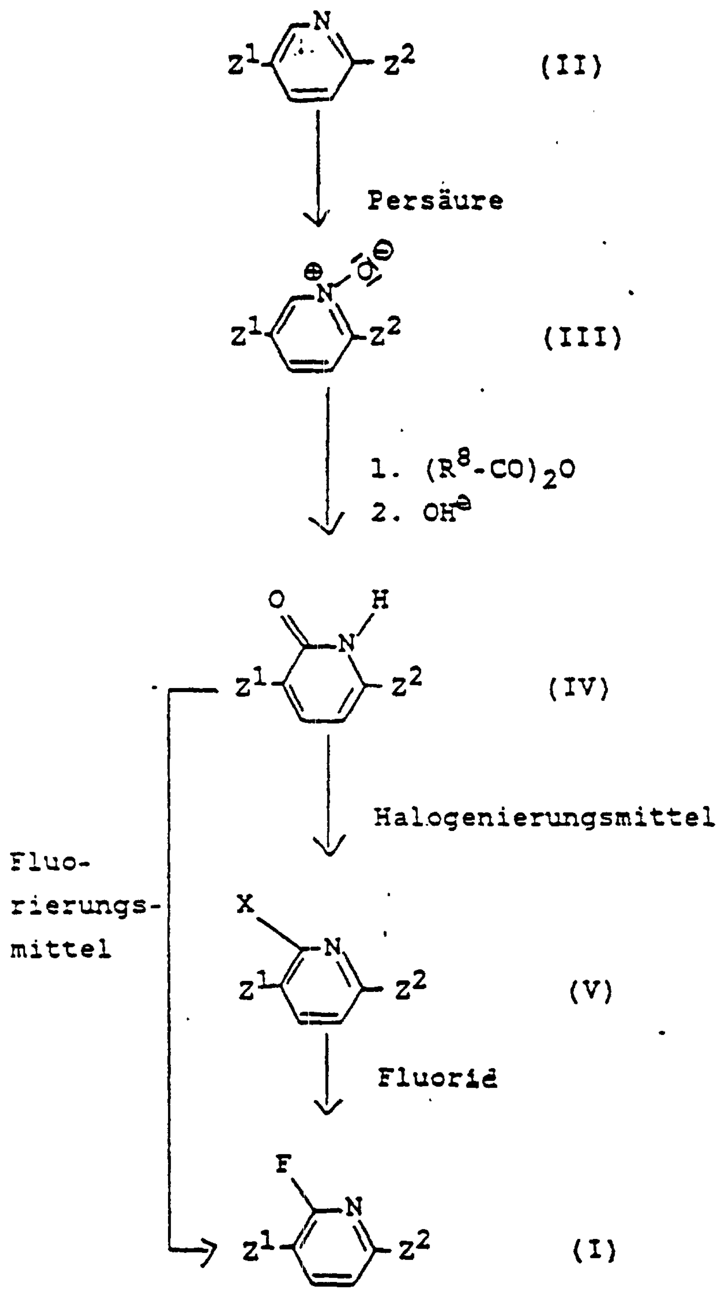


$Z^1 = R^1(-A^1)_k(-M^1)_l(-A^2)_m(-M^2)_n-$

$Z^2 = (-M^3)_o(-A^3)_p(-M^4)_q(-A^4)_r-R^2$

X = Cl, Br, I

$R^8$ = geradkettiges oder verzweigtes Alkyl C-Atomen.

Die erste Ausgangsverbindung des in den Schemata 2 bis 5 skizzierten Verfahrens B ist das kommerziell erhältliche 2,6-Difluorpyridin (Formel (VI)). Der Austausch eines Fluorsubstituenten in (VI) gegen eine Gruppierung der allgemeinen Formel $Z^2$ $(-M^3)_o(-A^3)_p(-M^4)_q(-A^4)_r-R^2$ durch Umsetzung mit einer Metallverbindung von $Z^2$, z.B. einer Lithium-, Natrium-, Kalium- oder Magnesiumverbindung bei Temperaturen zwischen-40 und 100°C, insbesondere zwischen -10 und 70°C in einem inerten Reaktionsmedium, z.B. Diethylether, Tetrahydrofuran, 1,4-Dioxan, Ethylenglykoldiethylether oder Diethylenglykoldiethylether führt zu Verbindungen der Formel (IX).

Die zweite Ausgangsverbindung des Verfahrens B ist das ebenfalls kommerziell erhältliche 2,6-Dibrompyridin (Formel (VII)). Durch die Umsetzung von 2,6-Dibrompyridin mit 1 bis 3, insbesondere 1,5 bis 2,5 Mol-Äquivalenten eines Fluorid-Reagenzes, wie z.B. Silberfluorid, Natriumfluorid, Kaliumfluorid oder Caesiumfluorid, bei Temperaturen zwischen 50 und 250°C, insbesonders zwischen 100 und 200°C, und einem Druck zwischen 50 und 300 mm Hg, insbesonders zwischen 100 und 200 mm Hg, unter Verwendung katalytischer Mengen (1 bis 20 Mol-Prozent, insbesonders 5 bis 15 Mol-Prozent) eines Komplexbildners, wie z.B. 18-Krone-6, Dibenzo-18-Krone-6 oder 1,10-Diaza-4,7,13,16,21,24-hexaoxabicyclo[8.8.8]-hexacosan wird 2-Brom-6-fluorpyridin (VIII) erhalten.

Die Kreuzkupplung von Verbindung (VIII) mit metallorganischen Derivaten von $Z^2$, z.B. Grignard-, Lithium- und Zinkderivaten, sowie Boronsäuren von $Z^2$ unter Verwendung von Übergangsmetallkatalysatoren, z.B. Dichloro [1,3-bis(diphenylphosphino)propan]nickel[II] und Tetrakis(triphenylphosphin)palladium[0] bei Temperaturen zwischen -40 und 200°C, insbesondere zwischen -10 und 100°C, in Reaktionsmedien, wie z.B. Benzol/Ethanol/Wasser für die Umsetzung mit Boronsäuren von $Z^2$ und z.B. Diethylether oder Tetrahydrofuran für die Umsetzung mit Grignard-, Lithium- und Zinkderivaten von $Z^2$, liefert ebenfalls Verbindungen des Typs (IX).

2-Fluorpyridine des Typs (IX) können durch Behandlung mit einer Lithiumverbindung, wie z.B. Lithiumalkylen oder Lithiumamiden, bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether, in 2-Fluor-3-lithiumpyridine der Formel (X) überführt werden. 3-Lithiumpyridine der allgemeinen Formel (X) sind der Umsetzung mit elektrophilen Verbindungen zugänglich, wodurch entweder direkt oder über weitere Zwischenstufen (Verbindungen der Formeln (XI), (XII), (XIII), (XIV), (XV) und (XVI) 2-Fluorpyridine der Formel (I) erhalten werden können.

So führt die Reaktion von Verbindungen des Typs (X) mit Halogniden, Nitrilen Carbonsäurehalogeniden und Formylmethylderivaten von $Z^3$ bei Temperaturen zwischen -40 und -100°C, insbesondere zwischen-60 und -80°C, in einem inerten Reaktionsmedium, z.B. Diethylether, Tetrahydrofuran oder Ethylenglykoldiethylether, direkt zu 2-Fluorpyridinen der Formel (I). Olefinische 2-fluorpyridine (I) lassen sich durch Hydrierung der olefinischen Doppelbindung nach an sich literaturbekannten Methoden (siehe z.B. Houben-Weyl, Methoden der Organischen Chemie, Georg Thieme Verlag, Stuttgart) in gesättigte Spezies (I) umwandeln.

Die Umsetzung von 2-Fluor-3-lithiumpyridinen (X) mit Halogenen, z.B. Chlor, Brom oder Jod bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether führt zu 2-Fluor-3-halogenpyridinen der Formel (XI). Durch Kreuzkupplung von Verbindungen des Typs (XI) mit metallorganischen Derivaten von $Z^1$, z.B. Grignard-, Lithium- und Zinkderivaten, sowie Boronsäuren von $Z^1$ unter Verwendung von Übergangsmetallkatalysatoren. z.B. Dichloro[1,3-bis(diphenylphosphino)propan]nickel[II] und Tetrakis(triphenylphosphin)palladium[0], bei Temperaturen zwischen -40 und 200°C, insbesondere zwischen -10 und 100°C, in Reaktionsmedien, wie z.B. Benzol/Ethanol/Wasser für die Umsetzung mit Boronsäuren von $Z^1$ und z.B. Diethylether oder Tetrahydrofuran für die Umsetzung mit Grignard-, Lithium- und Zinkderivaten von $Z^1$, erhält man 2-Fluorpyridine (I).

2-Fluor-3-lithiumpyridine (X) führen nach der Behandlung mit Kohlendioxid bei Temperaturen zwischen -40 und -100°C, insbesondere zwischen -60 und -80°C, in einem inerten Reaktionsmedium, z. B. Diethylether, Tetrahydrofuran oder Ethylenglykoldiethylether zu 2-Fluor-3-pyridincarbonsäuren der allgemeinen Formel (XII). Die Spezies (XII) können nach an sich literaturbekannten Methoden (siehe z.B. Houben-Weyl, Methoden der Organischen Chemie, Georg Thieme Verlag, Stuttgart), entweder direkt durch Veresterung mit Alkoholen von $Z^3$ unter Zuhilfenahme geeigneter Kondensationsmittel, z.B. Carbodiimiden, zu 2-Fluorpyridinen (I), oder nach Reduktion zu 2-Fluor-3-hydroxymethylpyridinen (XIII) mit geeigneten Reduktionsmitteln, z.B. komplexen Hydriden, durch Veresterung mit Carbonsäuren bzw. Carbonsäurehalogeniden von $Z^3$ oder Veretherung mit Alkoholen bzw. Halogeniden von $Z^3$ zu Verbindungen der Formel (I) umgesetzt werden.

Durch Reaktion von 2-Fluor-3-lithiumpyridinen (X) mit Ameisensäureamiden bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether werden 2-Fluor-3-formylpyridine (XIV) erhalten, welche nach der sauer katalysierten Acetalisierung mit 2-$Z^4$-1,3-Propandiolen nach an sich literaturbekannten Methoden-(siehe z.B. Houben-Weyl, Methoden der Organischen Chemie, Georg Thieme Verlag, Stuttgart) 2-Fluorpyridine des Typs (I) liefern.

Bei der sukzessiven Behandlung der 2-Fluor-lithiumpyridine (X) mit Borsäuretrialkylestern bei Temperaturen zwischen -40 und -100°C, insbesondere zwischen -60 und -80°C, und wäßriger Säure bei Temperaturen zwischen -10 und 50°C, insbesondere zwischen 10 und 30°C in einem inerten Reaktionsmedium, z.B. Diethylether, Tetrahydrofuran oder Ethylenglykoldiethylether, werden 2-Fluor-3-pyridinboronsäuren der Formel (XIV) erhalten.

Die Boronsäuren (XV) können Kupplungsreaktion mit Halogeniden von $Z^3$ unter Verwendung eines Übergangsmetallkatalysators, z.B. Tetrakis(triphenylphosphin)palladium[0] bei Temperaturen zwischen 30 und 200°C, insbesondere zwischen 50 und 100°C, in Reaktionsmedien, wie z.B. Benzol/Ethanol/Waser, zur Darstellung von Verbindungen des Typs (I) unterworden werden.

2-Fluorpyridine (I) werden aus den Boronsäuren (XV) desweiteren durch deren Veresterung mit 2-$Z^4$-1,3-Propandiolen nach an sich literaturbekannten Methoden (siehe z.B. Houben-Weyl, Methoden der Organischen Chemie, Georg Thieme Verlag, Stuttgart) erhalten.

Die Oxidation der Boronsäuren (XV) mit Peroxiden, z.B. Wasserstoffperoxid, bei Temperaturen zwischen 10 und 100°C, insbesondere zwischen 30 und 70°C, in Reaktionsmedien, wie z.B. Diethylether oder Tetrahydrofuran, führt zu den 2-Fluor-3-hydroxypyridinen (XVI), welche sich nach an sich literaturbekannten Methoden (siehe z.B. Houben-Weyl, Methoden der Organischen Chemie, Georg Thieme Verlag, Stuttgart) durch Veresterung mit Carbonsäuren bzw. Carbonsäurehalogeniden von $Z^3$ oder durch Veretherung mit Alkoholen bzw. Halogeniden von $Z^3$ in 2-Fluorpyridine der allgemeinen Formel (I) überführen lassen.

Schema 2 (Verfahren B):

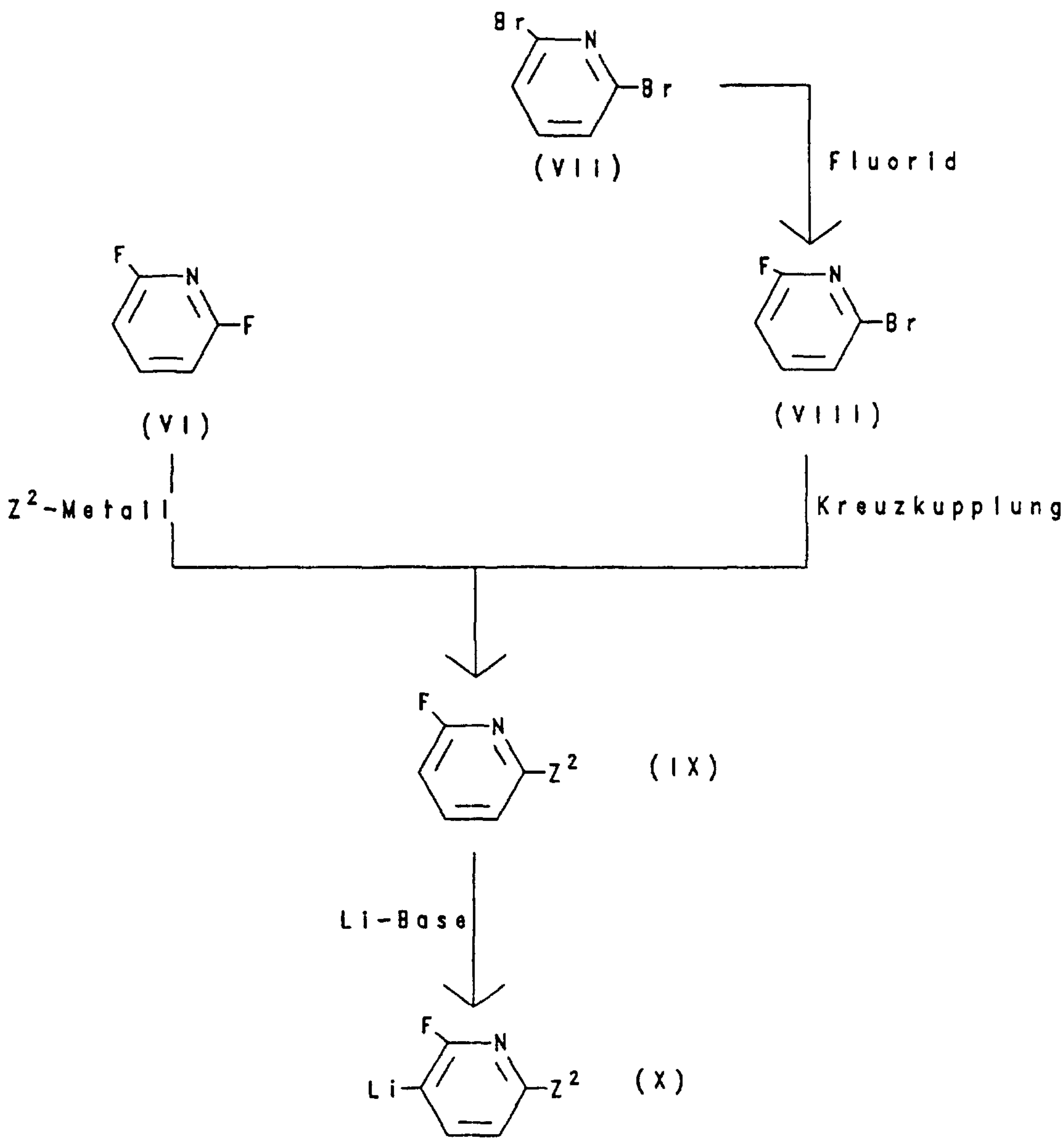

(VII)
(VI)
(VIII)
(IX)
(X)

Schema 3 (Verfahren B):

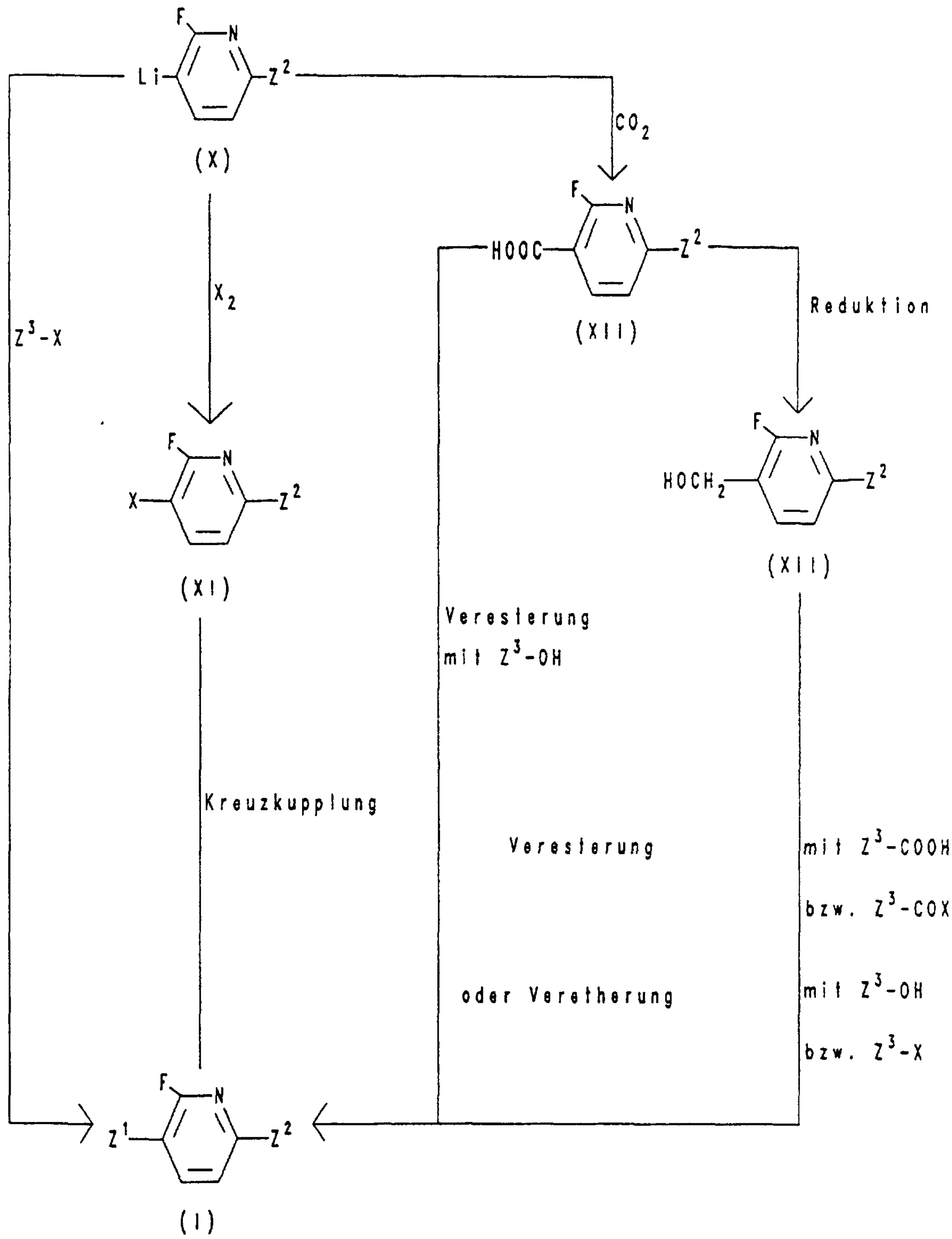


$Z^1$, $Z^2$, X siehe Schema 1

$Z^3 = R^1(-A^1)_k(-M^1)_l(-A^2)_m-$

Schema 4 (Verfahren B):

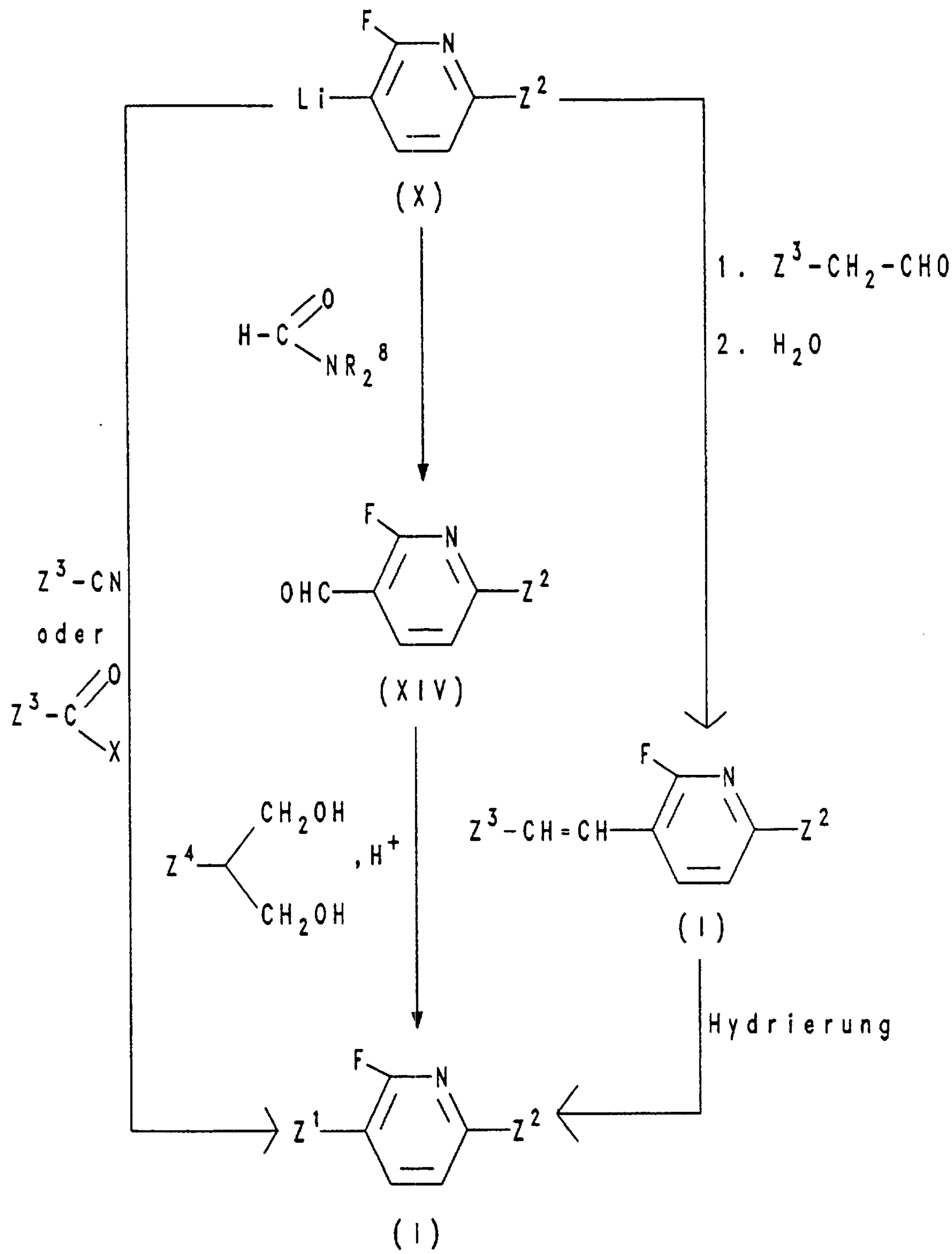


$Z^1$, $Z^2$, $Z^3$, $R^8$, X siehe Schemata 1 und 3

$Z^4 = R^1(-A^1)_k(-M^1)_l$-

Schema 5 (Verfahren B):

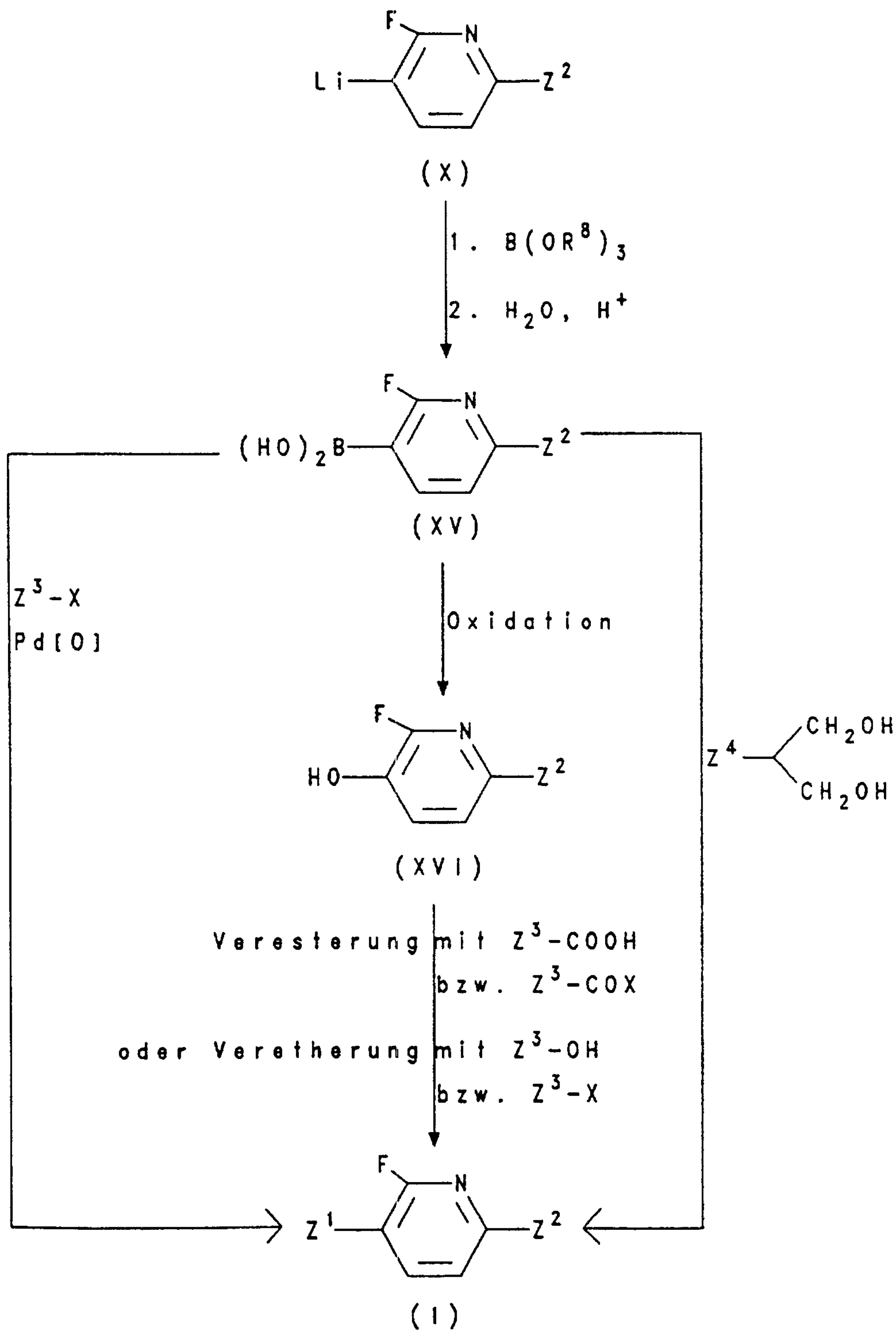


(X)

(XV)

(XVI)

(I)

$Z^1$, $Z^2$, $Z^3$, $Z^4$, $R^8$, X siehe Schemata 1, 3 und 4

Die Erfindung wird durch die nachfolgenden Beispiele näher erläutert:

Beispiel 1 Referenz

2-Fluor-3-octyl-6-(4-octyloxyphenyl)pyridin

Darstellung nach Verfahren B:

31,38 g (110,0 mmol) 1-Brom-4-octyloxybenzol in 80 ml Benzol und 81 ml (130,0 mmol) einer 1,6 molaren n-Butyllithiumlösung in Hexan werden unter Argon 4 h bei Raumtemperatur gerührt. Das als weißer Niederschlag entstandene 4-Octyloxyphenyllithium wird unter Argon abfiltriert, zweimal mit 20 ml n-Hexan gewaschen, im Vakuum getrocknet und als Lösung in 60 ml Tetrahydrofuran langsam bei 0°C unter Argon zu einer Lösung von 11,51 g (100,0 mmol) 2,6-Difluorpyridin in 80 ml Tetrahydrofuran getropft. Nach einstündiger Reaktionszeit bei 0°C wird mit Natriumchloridlösung versetzt, in Ether aufgenommen, die organische Phase mit Natriumchloridlösung gewaschen, über Natriumsulfat getrocknet, filtriert, vom Lösungsmittel befreit und chromatographisch (Kieselgel/Hexan: Essigester (9:1)) gereinigt. Es werden 4,00 g 2-Fluor-6-(4-octyloxyphenyl)pyridin erhalten.

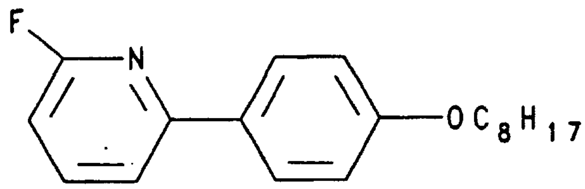

0,74 g (7,3 mmol) Diisopropylamin in 30 ml Tetrahydrofuran werden bei 0°C mit 4,56 ml (7,3 mmol) einer 1,6 molaren n-Butyllithiumlösung in Hexan für 1 h unter Argon gerührt. Nach Abkühlen auf -78°C werden 2,00 g (6,64 mmol) 2-Fluor-6-(4-octyloxyphenyl)pyridin in 30 ml Tetrahydrofuran so zugetropft, daß die Temperatur -70°C nicht übersteigt. Nach 4 h bei -78°C werden 1,35 g (7,00 mmol) 1-Bromoctan in 10 ml Tetrahydrofuran ebenfalls unter Einhaltung einer Temperatur ≤-70°C zugetropft. Das Reaktionsgemisch wird über Nacht gerührt, wobei es sich auf Raumtemperatur erwärmt, mit 5 ml Wasser versetzt und zur Trockne eingeengt. Nach Aufnehmen in Ether wird zweimal mit Wasser gewaschen, über Natriumsulfat getrocknet, filtriert, das Lösungsmittel abdestilliert, chromatographisch gereinigt (Kieselgel/Hexan: Essigester (9:1)) und aus Acetonitril umkristallisiert. Es werden 0,10 g 2-Fluor-3-octyl-6-(4-octyloxyphenyl)pyridin erhalten, welches in seinen physikalischen Eigenschaften mit dem nach Beispiel 1 hergestellten Produkt übereinstimmt.

Beispiel 2 Referenz

2-Fluor-3-octyloxy-6-(4-octyloxyphenyl)pyridin

Darstellung nach Verfahren B:

Zu 2,36 g (7,8 mmol) 2-Fluor-6-(4-octyloxyphenyl)-pyridin (hergestellt wie in Beispiel 2 beschrieben) in 180 mol Tetrahydrofuran werden bei -78°C 4,3 ml (8,6 mmol) einer 2-molaren Lithiumdiisopropylamidlösung in Tetrahydrofuran/Hexan/Ethylbenzol getropft und 4 h gerührt. Anschließend werden 2,95 g (17,2 mmol) Triisopropylborat in 10 ml Tetrahydrofuran bei -78°C zugetropft und das Reaktionsgemisch über Nacht gerührt, wobei es sich auf Raumtemperatur erwärmt. Nach Zugabe von 12,0 ml 10 %iger Salzsäure und 1 h Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Der Rückstand wird in 40 ml Ether aufgenommen und zum Sieden erhitzt. Es werden 8,03 g (23,5 mmol) einer 10 %igen Wasserstoffperoxidlösung zugetropft und 4 h unter Rückfluß gekocht. Nach Abtrennen der Etherphase wird die wäßrige Phase mit Ether ausgeschüttelt. Die vereinigten Etherphasen werden mit Natriumsulfitlösung ausgeschüttelt, mit Natriumchloridlösung gewaschen, über Natriumsulfat getrocknet, filtriert und zur Trockne eingeengt. Nach chromatographischer Reinigung (Kieselgel/Hexan: Essigester (7:3)) werden 1,13 g 2-Fluor-3-hydroxy-6-(4-octyloxyphenyl)pyridin erhalten.

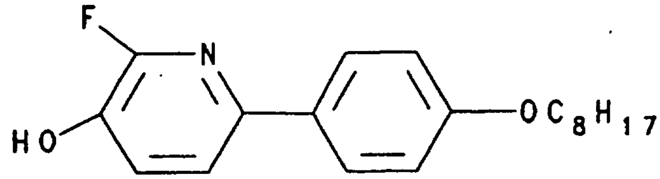

Zu 3,00 g (11,5 mmol) Triphenylphosphin in 50 ml Tetrahydrofuran werden bei 0°C 2,00 g (11,5 mmol) Azodicarbonsäurediethylester getropft und 30 min bei Raumtemperatur gerührt. Anschließend werden 2,42 g (7,7 mmol) 2-Fluor-3-hydroxy-6-(4-octyloxyphenyl)pyridin und 1,00 g (7,7 mmol) 1-Octanol zugegeben. Nach einer Reaktionszeit von 18 h bei Raumtemperatur wird das Lösungsmittel abdestilliert und der Rückstand chromatographisch (Kieselgel/Hexan: Essigester (95:5) gereinigt. Nach Umkristallisation aus Acetonitril werden 1,38 g 2-Fluor-3-octyloxy-6-(4-octyloxyphenyl)pyridin erhalten.

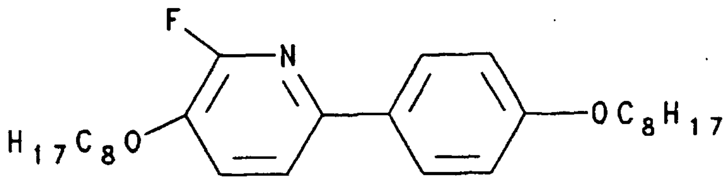

Die Verbindung zeigt die Phasenfolge:

X 81 $S_C$ 88 I

## Patentansprüche

1. Fluorpyridinverbindung der allgemeinen Formel I

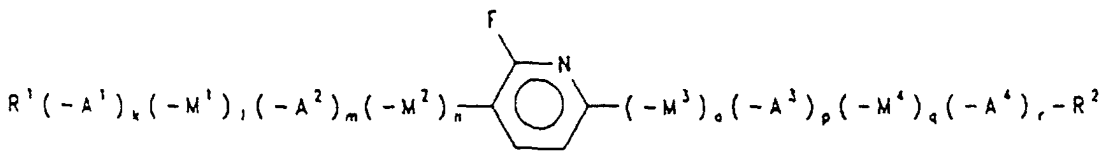

(I)

in der die Symbole folgende Bedeutung haben
$R^1$, $R^2$ unabhängig voneinander H,F,Cl,CN, $-CF_3$,$-OCF_3$,$-OCHF_2$ oder geradkettiges oder verzweigtes (mit oder ohne asymmetrisches C-Atom) Alkyl mit 1 bis 16 Atomen, wobei auch eine oder zwei nicht benachbarte $-CH_2$-Gruppen durch -O-, -CO-,-CO-O-,-O-CO-, -O-CO-O-,-CH = CH-,-C ≡C-, oder $-Si(CH_3)_2$- ersetzt sein können, und wobei auch ein oder mehrere H-Atome des Alkylrestes durch F substituiert sein können, oder eine der nachfolgenden chiralen Gruppen:

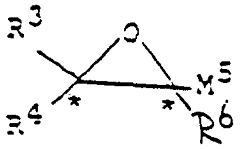 , 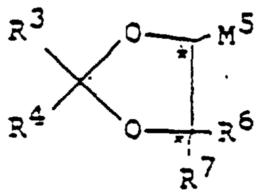 , 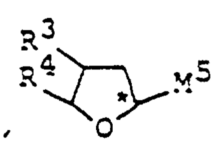 , 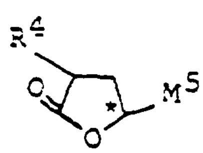 ,

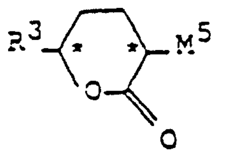 , 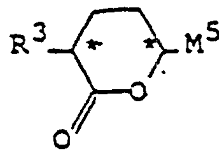 , 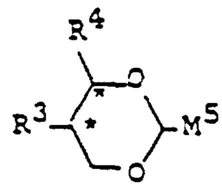 , 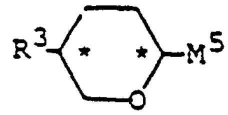

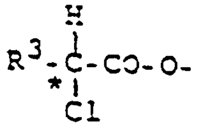 , 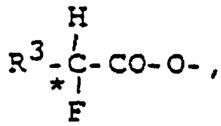 , 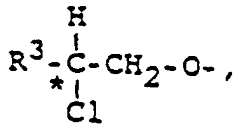 , 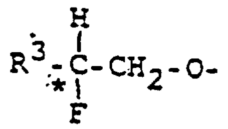 ,

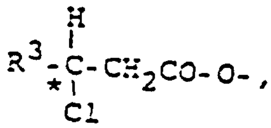 , 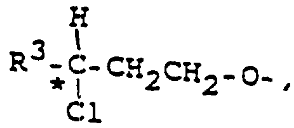 ,

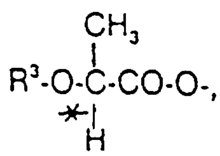 , 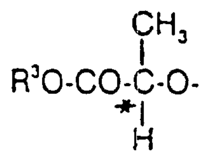

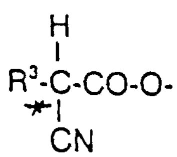 , 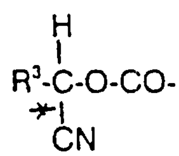

$A^1$, $A^2$, $A^3$, $A^4$ sind gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome durch F ersetzt sein können, trans-1,4-Cyclohexylen, bei dem ein oder zwei H-Atome durch CN ersetzt sein können, (1,3,4)-Thiadiazol-2,5-diyl, 1,3-Dioxan-2,5-diyl, Naphthalin-2,6-diyl, Bicyclo[2.2.2.]octan-1,4diyl oder 1,3-Dioxaborinan-2,5-diyl;

$M^1$, $M^2$, $M^3$, $M^4$ gleich oder verschieden -O-, -CO-, -CO-O-, -O-CO-, -O-CO-O-, $-CH_2-O-$, $-O-CH_2-$, $-CH_2-CH_2-$, -CH =CH- oder -C≡C-;

$R^3$, $R^4$, $R^6$, $R^7$ unabhängig voneinander H oder geradkettiges oder verzweigtes Alkyl mit 1 bis 16 C-Atomen oder $R^3$ und $R^4$ zusammen auch $-(CH_2)_4-$ oder $-(CH_2)_5-$, wenn als Substituenten an ein Dioxolan System gebunden;

$M^5$ $-CH_2-O-$, -CO-O-, $-O-CH_2-$, -O-CO- oder eine Einfachbindung

k, l, m, n, o, p, q, r Null oder Eins, unter der Bedingung, daß die Summe

k + m + p + r kleiner 4 und größer Null ist,

mit der Maßgabe, daß eine der beiden Gruppen

$R^1$ und $R^2$ die Gruppe

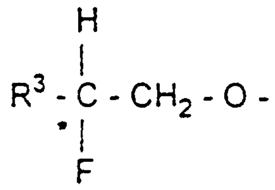

ist.

2. Flüssigkristalline Mischung, enthaltend mindestens eine Fluorpyridinverbindung der Formel (I) gemäß Anspruch 1.

3. Flüssigkristalline Mischung nach Anspruch 2, **dadurch gekennzeichnet, daß** sie aus 2 bis 20 Komponenten besteht.

4. Flüssigkristalline Mischung nach Anspruch 2 oder 3, **dadurch gekennzeichnet, daß** sie 0,1 bis 70 Mol-% an einem oder mehreren Fluorpyridinen der Formel (I) gemäß Anspruch 1 enthält.

5. Flüssigkristalline Mischung nach einem oder mehreren der Ansprüche 2 bis 4, **dadurch gekennzeichnet, daß** sie ferroelektrisch ist.

6. Ferroelektrische Schalt- und Anzeigevorrichtung, enthaltend Trägerplatten, Elektroden, mindestens einen Polarisator, mindestens eine Orientierungsschicht sowie ein flüssigkristallines Medium, **dadurch gekennzeichnet, daß** das flüssigkristalline Medium eine Flüssigkristallmischung nach Anspruch 5 ist.

## Claims

1. A fluoropyridine compound of the formula I

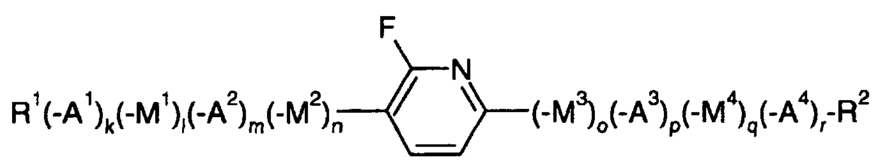

in which the symbols have the following meaning:

$R^1$, $R^2$, independently of one another, are H, F, Cl, CN, $-CF_3$, $-OCF_3$, $-OCHF_2$ or straight-chain or branched (with or without the inclusion of an asymmetric carbon atom) alkyl having 1 to 16 atoms, it also being possible for one or two not neighboring $-CH_2-$ groups to be replaced by -O-, -CO-, -CO-O-, -O-CO-, -O-CO-O-, -CH=CH-, -C≡C- or $-Si(CH_3)_2-$, wherein one or more H atoms of the alkyl residue may be substituted by F or are one of the following chiral groups:

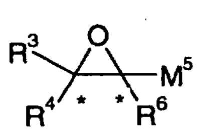 , 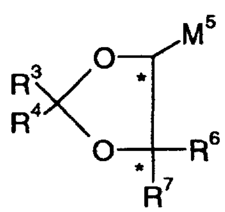 , 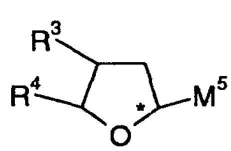 , 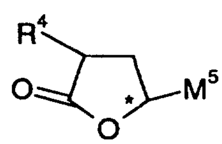 ,

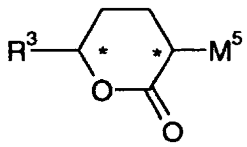, 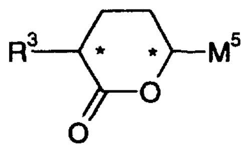, 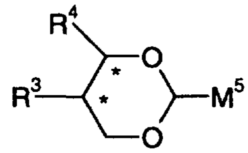, 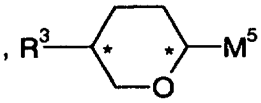,

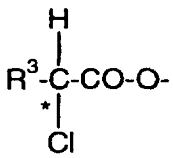, 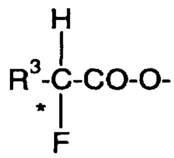, 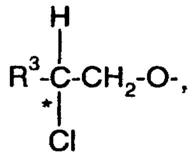 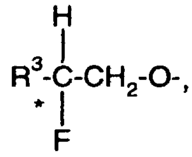

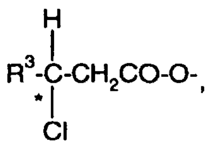 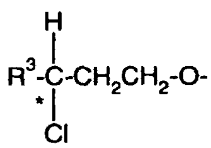,

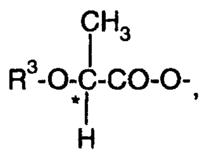 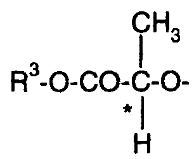,

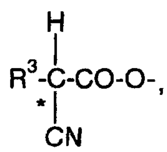 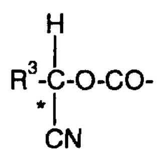,

$A^1$, $A^2$, $A^3$, $A^4$, identical or different, are 1,4-phenylene, pyrazine-2,5-diyl, pyridazine-3,6-diyl, pyridine-2,5-diyl, pyrimidine-2,5-diyl, it being possible for one or two hydrogen atoms to be replaced by F, trans-1,4-cyclohexylene in which one or two H atoms may be replaced by CN, 1,3,4-thiadiazole-2,5-diyl, 1,3-dioxane-2,5-diyl, naphthalene-2,6-diyl, bicyclo[2.2.2]octane-1,4-diyl or 1,3-dioxaborinane-2,5-diyl;

$M^1$, $M^2$, $M^3$, $M^4$, identical or different, are -O-, -CO-, -CO-O-, -O-CO-, -O-CO-O-, $-CH_2-O-$, $-O-CH_2-$, $-CH_2-CH_2-$, -CH=CH- or -C≡C-;

$R^3$, $R^4$, $R^6$, $R^7$, independently of one another, are H or straight-chain or branched alkyl having 1 to 16 carbon atoms or $R^3$ and $R^4$ together are also $-(CH_2)_4-$ or $-(CH_2)_5-$ if bound as substituents to a dioxolane system;

$M^5$ is $-CH_2-O-$, -CO-O-, $-O-CH_2-$, -O-CO- or a single bond;

k, l, m, n, o, p, q, r are zero or one, with the proviso that the sum of k + m + p + r is less than 4 and greater than zero,

with the proviso that one of the two groups

$R^1$ and $R^2$ is the group

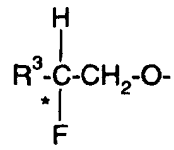

**2.** A liquid crystal mixture comprising at least one fluoropyridine compound of the formula (I) as claimed in claim 1.

**3.** A liquid crystal mixture as claimed in claim 2, which consists of from 2 to 20 components.

**4.** A liquid crystal mixture as claimed in claim 2 or 3, which comprises from 0.1 to 70 mol%, of one or more fluoropyridines of the formula (I) as claimed in claim 1.

**5.** A liquid crystal mixture as claimed in one or more of claims 2 to 4, which is ferroelectric.

**6.** A ferroelectric switching and display device, comprising base plates, electrodes, at least one polarizer, at least one orientation layer and a liquid-crystalline medium, wherein the liquid-crystalline medium is a liquid crystal mixture as claimed in claim 5.

## Revendications

**1.** Composé de la fluoropyridine de formule générale I

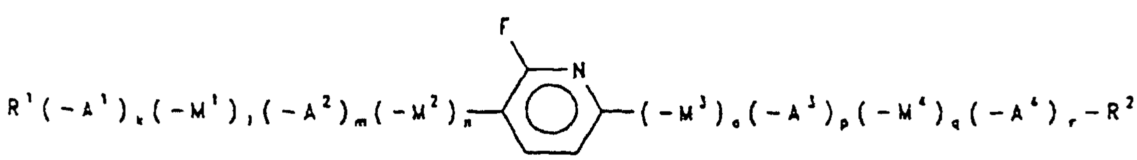

(I)

dans laquelle les symboles possèdent les significations suivantes :

$R^1$, $R^2$ indépendamment l'un de l'autre représentent H, F, Cl, CN, $-CF_3$, $-OCF_3$, $-OCHF_2$ ou un groupe alkyle linéaire ou ramifié (avec ou sans atome de carbone asymétrique) présentant 1 à 16 atomes, un ou deux groupes $-CH_2-$ non adjacents pouvant être remplacés par -O-, -CO-, -CO-O-, -O-CO-, -O-CO-O-, -CH=CH-, -C≡C- ou $-Si(CH_3)_2-$ et un ou plusieurs atomes d'hydrogène du reste alkyle pouvant être substitués par des atomes de fluor ou un des groupes chiraux suivants :

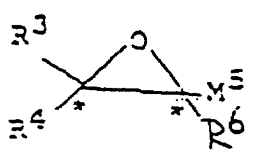 , 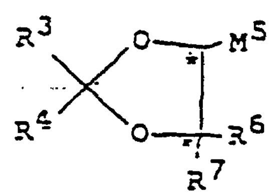 , 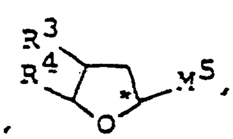 , 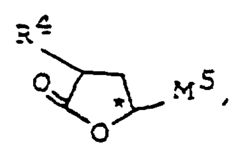 ,

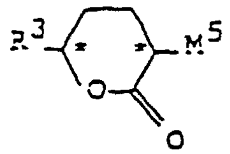 , 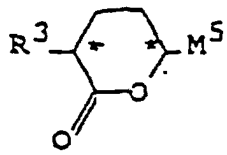 , 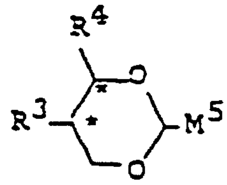 , 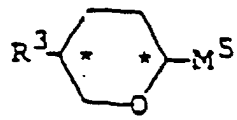

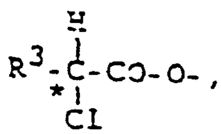 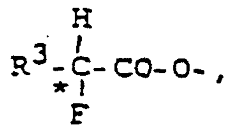 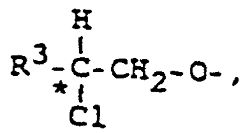 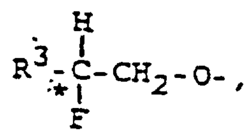

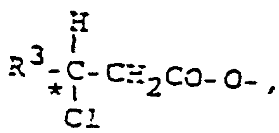 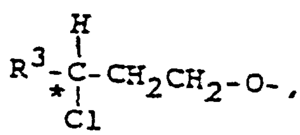

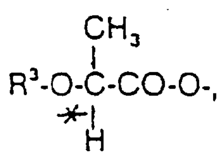 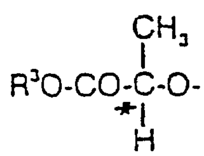

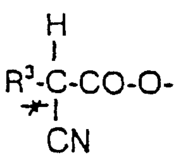 , 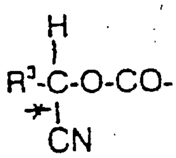

| | |
|---|---|
| $A^1$, $A^2$, $A^3$, $A^4$ | sont identiques ou différents et représentent un groupe 1,4-phénylène, pyrazin-2,5-diyle, pyridazine-3,6-diyle, pyridine-2,5-diyle, pyrimidine-2,5-diyle, un ou deux atomes d'hydrogène pouvant être remplacés par des atomes de fluor, trans-1,4-cyclohexylène, dans lequel un ou deux atomes d'hydrogène peuvent être remplacés par un groupe CN, (1,3,4)-thiadiazol-2,5-diyle, 1,3-dioxane-2,5-diyle, naphtalène-2-6-diyle, bicyclo[2.2.2.]octane-1,4-diyle ou 1,3-dioxaborinane-2,5-diyle ; |
| $M^1$, $M^2$, $M^3$, $M^4$ | sont identiques ou différents et représentent un groupe -O-, -CO-, -CO-O-, -O-CO-, -O-CO-O-, $-CH_2-O-$, $-O-CH_2-$, $-CH_2-CH_2-$, -CH=CH- ou -C≡C- ; |
| $R^3$, $R^4$, $R^6$, $R^7$ | indépendamment les uns des autres représentent un atome d'hydrogène ou un groupe alkyle linéaire ou ramifié présentant 1 à 16 atomes ou $R^3$ et $R^4$ conjointement forment aussi un groupe $-(CH_2)_4-$ ou $-(CH_2)_5-$ lorsqu'ils sont liés en tant que substituants à un système dioxolane ; |
| $M^5$ | représente un groupe $-CH_2-O-$, -CO-O-, $-O-CH_2-$, -O-CO ou une liaison simple ; |
| k, l, m, n, o, p, q, r | valent zéro ou un, à condition que la somme de k + m + p + r soit inférieure à 4 et supérieure à zéro, |

à condition qu'un des deux groupes

$R^1$ et $R^2$ représente le groupe

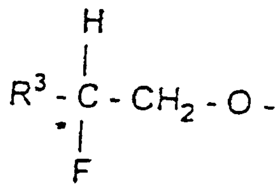

**2.** Mélange de cristaux liquides renfermant au moins un composé de la fluoropyridine de formule (I) selon la revendication 1.

**3.** Mélange de cristaux liquides selon la revendication 2, **caractérisé en ce qu'**il est constitué par 2 à 20 constituants.

**4.** Mélange de cristaux liquides selon la revendication 2 ou 3, **caractérisé en ce qu'**il renferme de 0,1 à 70 % molaires d'une ou plusieurs fluoropyridines de formule (I) selon la revendication 1.

**5.** Mélange de cristaux liquides selon une ou plusieurs des revendications 2 à 4, **caractérisé en ce qu'**il est ferroélectrique.

**6.** Dispositif d'affichage et de commutation ferroélectrique comprenant des plaques de support, des électrodes, au moins un polariseur, au moins une couche d'orientation ainsi qu'un milieu de cristaux liquides, **caractérisé en ce que** le milieu de cristaux liquides est un mélange de cristaux liquides selon la revendication 5.